# FASCICULE DE BREVET EUROPEEN

(11) **EP 0 736 102 B1**
(45) Date de publication et mention de la délivrance du brevet: **26.04.2000**
(21) Numéro de dépôt: 95904576.6
(22) Date de dépôt: 20.12.1994
(51) Int. Cl.: C12P 21/06, C07K 1/00, C07K 2/00, C07K 14/47

(54) **PROCEDE D'OBTENTION DE PRODUITS PEPTIDIQUES**
Methode zur Herstellung von Peptidprodukten
METHOD FOR PREPARING PEPTIDE PRODUCTS

(30) Priorité: 23.12.1993 FR 9315764
(43) Date de publication de la demande: 09.10.1996
(73) Titulaire: INSTITUT NATIONAL DE LA RECHERCHE AGRONOMIQUE, 75338 Paris Cédex 07 (FR)
(72) Inventeur: CHOBERT, Jean-Marc, F-44300 Nantes (FR); BRIAND, Loic, F-44300 Nantes (FR); HAERTLE, Tomasz, F-44240 La-Chapelle-sur-Erdre (FR)
(74) Mandataire: Phélip, Bruno
(86) Numéro de dépôt international: FR9401500
(87) Numéro de publication internationale: WO9517518

(56) Documents cités:
- EP-A- 0 047 879
- EP-A- 0 088 398
- WO-A-92/15279
- WO-A-93/18180
- CHEMICAL ABSTRACTS, vol. 116, no. 19, 11 Mai 1992, Columbus, Ohio, US; abstract no. 192692k, 'Esterification of food proteins : characterization of the derivatives by a colorimetric method and by electrophoresis' page 582 ; & SCI. ALIMENTS, vol.11, no.4, 1991 pages 641 - 652 BERTRAND-HARB, C. ET AL.

## Description

La présente invention concerne un procédé d'obtention de produits peptidiques à partir d'un substrat d'origine animale ou végétale contenant au moins une protéine ou un peptide, tel que défini dans les revendications.

Selon la présente description l'expression "produits peptidiques" définit des populations peptidiques très variées pouvant contenir diverses entités telles que des peptides, fractions peptidiques, acides aminés, estérifiés et non estérifiés, et leurs mélanges.

Les protéines d'origine animale ou végétale, et les peptides issus de ces protéines, sont utilisés très couramment dans tout genre d'industrie (pharmaceutique, chimique, agro-alimentaire, cosmétologique ...) en raison de leurs propriétés chimiques ou physico-chimiques.

Les hydrolyses enzymatiques des protéines laitières donnent naissance à des peptides aux propriétés biologiques variées. Il en est ainsi des peptides opiacés (Zioudrou C., Streaty R.A. et Klee W.A. (1979), J.Biol Chem. **254**, 2446-2449 ; Brantl V., Teschemacher H., Henschen A. et Lottspeich F. (1981) Life Sci. **28**, 1903-1909 ; Yoshikawa M., Yoshimura T. et Chiba H. (1984), Agric. Biol. Chem. **48**, 3185-3187; Loukas S., Varoucha D., Zioudrou C., Streaty R.A. et Klee W.A. (1983), Biochemistry **22**, 4567-4573 ; Chiba H., Tani F. et Yoshikawa M. (1989) J.Dairy Res.,**56**, 363-366 ; Fukudome S.I. et Yoshikawa M. (1992), FEBS Lett **296**, 107-111 ; Antila P., Paakkari I., Järvinen A., Mattila M.J., Laukkanen M., Pihlanto-Leppälä A., Mäntsälä P. et Hellman J. (1991) Int. Dairy Journal **1**, 215-229 ; Meisel H. et Frister H. (1989), J.Dairy Res. **56**, 343-349).

On a également isolé des peptides antihypertenseurs (Maruyama S., Mitachi H., Tanaka H., Tomizuka N. et Suzuki H. (1987) Agric. Biol. Chem. **51**, 1581-1586 ; Komura M., Nio N., Kubo K., Minoshima Y., Munekata E. et Ariyoshi Y. (1989) Agric. Biol. Chem. **53**, 2107-2114), ainsi que des peptides immunostimulants (Berthou J., Migliore-Samour D., Lifchitz A., Delettré J., Floc'h F. et Jollès P. (1987), FEBS Lett. **218(1)**, 55-58 ; Migliore Samour D., Floc'h F. et Jollès P. (1989) J.Dairy Res. **56**, 357-362), ou des peptides antithrombotiques (Fiat A.M., Levy-Toledano S.P., Ceen J. et Jollès P. (1989) J. Dairy Res. **56**, 351-355).

D'autre part, les peptides issus des protéines du lait offrent également des propriétés physicochimiques et interfaciales intéressantes (Fox P.F. et Mulvihill D.M. (1983), dans Proceedings of IDF symposium, Holsingor, Denmark, 188-259 ; Chobert J.M., Bertrand-Harb C. et Nicolas M.G. (1988a), J.Agric. Food Chem. **36**, 883-892 ; Chobert J.M., Sitohy, M.Z. et Whitaker, J.R. (1988b) J.Agric. Food Chem. 36, 220-224 ; Chobert J.M., Bertrand-Harb C., Dalgalarrondo M. et Nicolas M.G. (1989a), J.Food Biochem. **13**, 335-352 ; Chobert J.M., Touati A., Bertrand-Harb C., Dalgalarrondo M. et Nicolas M.G. (1989b), J.Food Biochem. **13**, 457-473 ; Vuillemard J.C., Gauthier S. et Paquin P. (1989 Lait **69**, 323-351 ; Turgeon L.S., Gauthier F.S., Mollé D. et Léonil J. (1992) J.Agric. Food Chem. **40**, 669-675).

Au niveau nutritionnel et thérapeutique, l'hydrolyse enzymatique permet de réduire l'allergénicité des protéines sériques (Jost R., Monti J.CV. et Pahud J.J. (1987) Food Technol. **41**, 118-121).

On peut citer également, à titre de référence bibliographique pertinente, le brevet EP-22019 qui décrit un hydrolysat enzymatique total de protéines du lactosérum et les applications de cet hydrolysat à titre de médicament et en nutrition thérapeutique.

Du point de vue physiologique, les hydrolysats de protéines de lactosérum ont la propriété de stimuler la croissance de la régénération de la peau (Wenner V. (1982) Lait **62**, 549-565).

Il est connu d'agir sur les protéines ou les peptides d'origine animale ou végétale pour modifier leurs propriétés, notamment par greffage de substituants.
En particulier le Document WO-A-9318180 décrit l'estérification de peptides d'origine animale ou végétale par un procédé dénomme "synthèse enzymatique d'esters alkyliques de peptides" nécessitant l'emploi d'enzymes possédant à la fois une activité protéolytique et estérolytique. Le procédé en question se caractérise par la mise en contact simultanée du substrat protéique avec l'enzyme et un alcool, aboutissant à la formation d'esters alkyliques de peptides.
D'autre part, le document EP-A-0 047 879 décrit un procédé de préparation de compositions protéiques modifiées par ajout d'un ou de plusieurs acides aminés. Ce procédé consiste à hydrolyser un substrat protéique par une enzyme, à ajouter le ou les acides aminés supplémentaires ayant été au préalable estérifiés, et enfin à faire travailler l'enzyme de telle sorte qu'elle permette la reconstitution de la protéine avec le ou les acides aminés supplémentaires.

La présente invention a pour objet un procédé consistant à estérifier une matière protéique d'origine animale ou végétale contenant au moins une protéine ou un peptide, puis à hydrolyser par voie enzymatique la matière protéique estérifiée, ledit procédé conduisant à une population peptidique nouvelle. La modification des protéines par estérification aboutit à une nouvelle famille de peptides aux propriétés physico-chimiques et biologiques originales.

Le procédé selon l'invention consiste à réaliser une réaction d'estérification d'au moins un groupement estérifiable de l'une au moins des protéines ou peptides contenus dans le substrat d'origine animale ou végétale, dans le but de modifier l'action ultérieure de l'enzyme (sites de coupure et cinétique de réaction).

A titre d'exemple non limitatif de substrat protéique ou peptidique susceptible d'être utilisé, on peut citer : les protéines laitières (caséines et protéines du lactosérum), le gluten de blé ou de maïs, la zéïne, les concentrats et isolats protéiques de soja, pois, féverole ... ou encore le collagène, la sérumalbumine, l'ovalbumine ...

Cette réaction d'estérification est conduite selon les procédures classiques, parfaitement connues de l'homme du métier (Voir par exemple le procédé d'estérification en milieu acide décrit par Fraenkel-Conrat H. et Olcott H.S. 1945., j. Biol. Chem. **161**, 259-269). Elle est de préférence réalisée sur au moins un groupement carboxylique de la protéine ; elle est avantageusement conduite en milieu acide au moyen d'un alcool (ou d'un dérivé activé de cet alcool obtenu selon les méthodes classiques bien connues de l'homme du métier).
Par exemple on propose l'utilisation d'un alcool aliphatique possédant entre un et cinq atomes de carbone (méthanol, éthanol ...).

Les conditions de mise en oeuvre de la réaction d'estérification (température, durée, pression, concentration en acide ...) sont fonction du taux d'estérification désiré et, plus généralement, des produits finaux que l'on désire obtenir.

La protéine estérifiée est ensuite soumise à l'action d'au moins une enzyme protéolytique, dans des conditions habituelles du domaine technique considéré, pour scinder la chaîne d'acides aminés (Voir par exemple l'ouvrage édité par A.Neuberger et K.Brocklehurst (1987), coll.New Comprehensive Biochemistry - Vol.16 - Hydrolytic Enzymes, pub. Elsevier, ou encore l'ouvrage édité par R.J. Beynon et J.S.Bond (1989), coll. The Practical Approach Series - Proteolytic enzymes, a practical approach-IRL PRESS). Dans cette étape, toute enzyme protéolytique et/ou peptidasique (protéase, peptidase), peut être utilisée (pepsine, papaïne, trypsine, chymosine, chymotrypsine, thermolysine ...) ; l'hydrolyse peut être réalisée par l'une de ces enzymes ou par une pluralité d'entre elles, successivement ou simultanément.

Selon une variante du procédé de l'invention, la réaction d'estérification est précédée par une hydrolyse partielle de la protéine, au moyen d'une ou de plusieurs enzymes protéolytiques ou peptidasiques. Le taux d'hydrolyse partielle est laissé à l'appréciation de l'opérateur qui aura une large latitude d'action en fonction des produits qu'il désire obtenir. La seule contrainte sera de ne pas obtenir une hydrolyse totale de la protéine ou du peptide.

De façon étonnante, le fait de faire précéder par une réaction d'estérification l'opération d'hydrolyse, permet d'augmenter ou de changer la sensibilité de la protéine à l'hydrolyse enzymatique. L'enzyme est "leurrée" par la modification de la protéine ; en fonction des produits utilisés et des conditions opératoires, par rapport a la protéine native, on peut obtenir de nouveaux sites de coupure et/ou supprimer certains sites traditionnels. Cette façon d'opérer permet l'obtention de sites de coupure atypiques qui conduisent à l'obtention de peptides nouveaux, dont certains au moins sont estérifiés.

La mise en oeuvre du procédé selon l'invention aboutit à un mélange de peptides ou d'acides aminés et de leurs esters. Ces peptides, acides aminés et esters correspondants peuvent ensuite être séparés et purifiés pour obtenir des populations homogènes.

Dans le cadre d'une estérification par un alcool aliphatique, on obtient une protéine estérifiée au niveau de son groupement carboxylique C-terminal et au niveau des groupements carboxyliques latéraux de certains des résidus aspartyls et/ou glutamyls. L'action ultérieure de l'enzyme protéolytique permet d'obtenir un peptide terminal dont le groupement carboxylique C-terminal est estérifié ; tous les autres peptides obtenus, estérifiés ou non, comportent un groupement carboxylique C-terminal non estérifié.

Cette substitution au niveau du groupe carboxyle permet de supprimer une charge négative sur la protéine ou le peptide obtenu (suppression de charge au-dessus du pK du COOH des acides aspartiques et glutamiques constitutifs). On augmente le point isoélectrique de la protéine ou du peptide ; on les rend plus basiques et plus hydrophobes ce qui entraîne une meilleure affinité et interaction avec les interfaces biologiques et artificielles chargées en majorité négativement.

Selon l'invention, le traitement de résidus d'acides aminés par estérification, suivi d'une protéolyse, permet de formuler de nouvelles protéines ou populations peptidiques. Les moyens à mettre en oeuvre sont simples, peu coûteux, non toxiques et préservent la majorité des valeurs nutritionnelles de la protéine.

Les peptides estérifiés obtenus ont des propriétés électrostatiques, une hydrophobicité et une amphiphilie différentes qui leur confèrent des propriétés interfaciales, physiologiques, biologiques et immunologiques particulières.

L'estérification peut aussi constituer un moyen simple pour augmenter la sensibilité d'une protéine structurée à une action enzymatique.

Les domaines d'application de l'invention sont variés ; les produits obtenus peuvent avantageusement être utilisés à titre d'ingrédients, d'additifs ou d'agents actifs dans des préparations alimentaires, pharmaceutiques ou cosmétologiques.
De telles applications sont similaires à celles déjà décrites pour les hydrolyses enzymatiques de protéines connues, telles qu'elles ont été rappelées dans les références bibliographiques citées au début de la description et illustrant l'art antérieur.

### Exemples

### Exemple 1 : Protéolyse pepsique de la bêta-lactoglobuline

La bêta-lactoglobuline de bovin (variant B) est préparée selon la méthode de Mailliart, P., Ribadeau Dumas, B. (1988, j. FOOD Sci. **53**, 743-745).

### Estérification

Les esters de bêta-lactoglobuline sont préparés en utilisant un procédé dérivé de celui décrit par Fraenkel-Conrat, H., Olcott, H.S. (1945, j. Biol. Chem. **161**, 259-268).
La bêta-lactoglobuline est mise en suspension dans l'éthanol pour obtenir une suspension à 2 %. Sous agitation, de l'acide chlorhydrique 12N est ajouté doucement pour obtenir une suspension de protéine-alcool 0,06-0,68N en acide. Cette préparation est placée sous agitation à 4°C pendant plusieurs jours en fonction du degré d'estérification désiré. Après séchage sous vide, les échantillons sont stockés à une température de -80°C. Un échantillon témoin est préparé de la même manière sans addition d'HCl.

### Analyse des protéines estérifiées

Pour déterminer le degré d'estérification de la bêta-lactoglobuline avec l'éthanol, on a utilisé la réaction colorée utilisant l'hydrochlorure d'hydroxylamine développée par Halpin, M.I., Richardson, T. (1945, j. Dairy Sci. **68**, 3189-3198), modifiée selon Bertrand-Harb,C., Chobert, J.M., Dufour, E., Haertle, T. (1991, Sci. Aliments **11**, 641-652).
Dans le cas présent, la bêta-lactoglobuline a été estérifiée à 40 %.

### Hydrolyse

Les échantillons de bêta-lactoglobuline et de bêtalactoglobuline estérifiée ont ensuite été hydrolysés par la pepsine (pepsine porcine : 3,200-4,500 BAEE U/mg de la société Sigma Chimie, St-QUENTIN FALLAVIER, FRANCE).
La pepsine (1 mg/ml de H₂O, en concentration initiale) est ajoutée dans un rapport enzyme/substrat protéique (E/S) de 2 %. Le mélange est placé à incubation à 37°C. Des échantillons sont prélevés après une, deux, quatre, vingt et quarante heures ; l'hydrolyse est stoppée par addition de 1,5 volume d'un tampon Tris-HCl 0,2M, pH8,0.

### Résultats

Les divers peptides obtenus ont été purifiés et identifiés par leur composition en acides aminés et leur séquence N-Terminale.

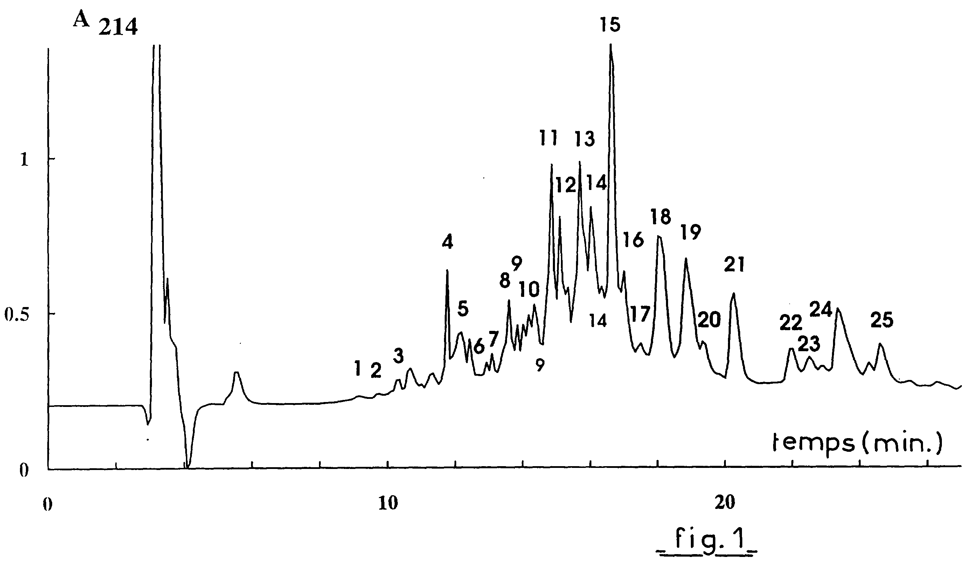

Le profil chromatographique CLHP de l'hydrolysat pepsique (après 40 heures d'hydrolyse) de la bêta-lactoglobuline éthylée est représenté sur la figure 1 (Colonne C₁₈, porosité 10 µm, longueur 25 cm, diamètre intérieur 0,4 cm de la SFCC SHANDON, GAGNY, FRANCE).

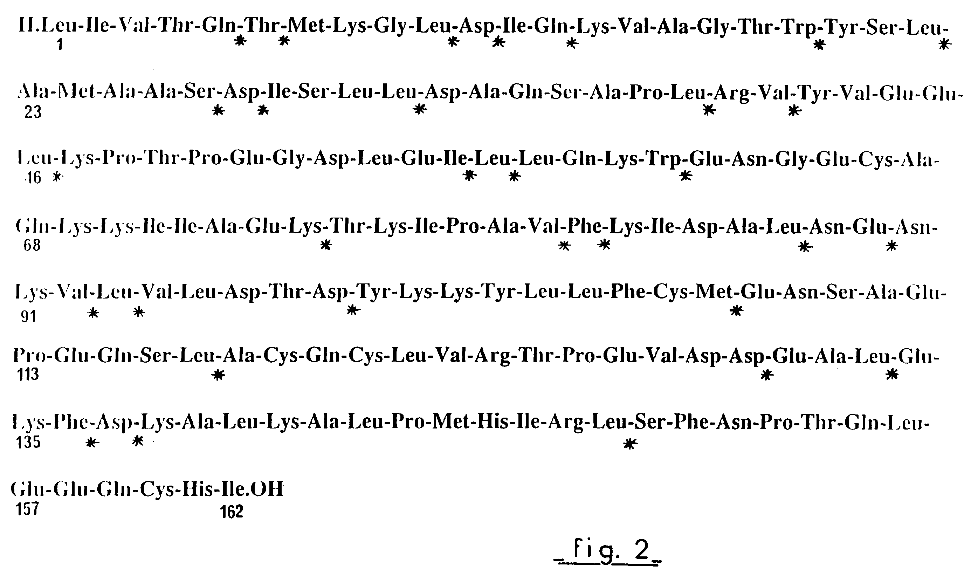

La structure primaire de la bêta-lactoglobuline B apparaît sur la figure 2, avec les sites de coupure pepsiques(*).

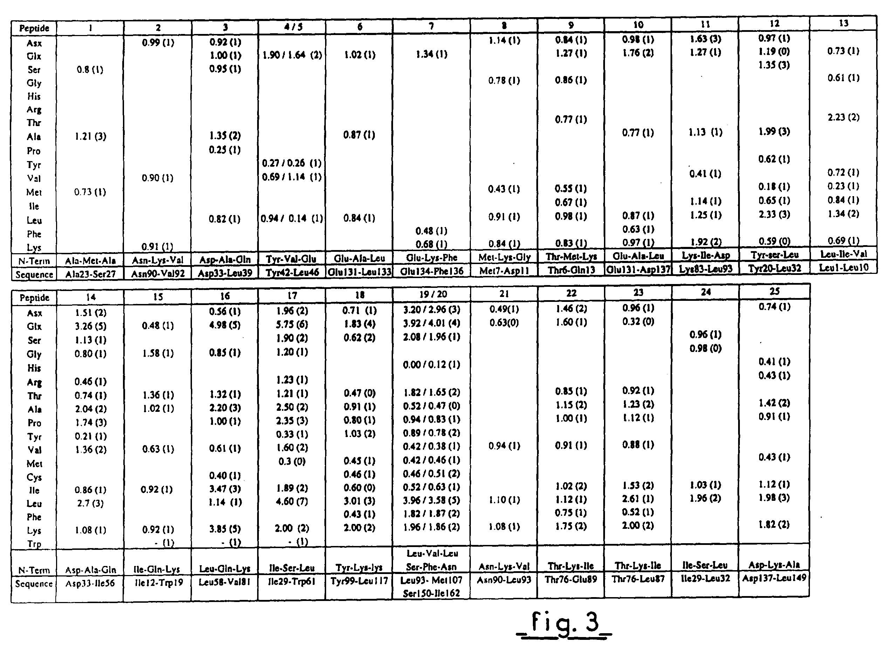

La figure 3 montre, sous forme de tableaux, la composition en acides aminés et séquences N-Terminales des peptides pepsiques de la bêta-lactoglobuline éthylée (ester).

L'étude cinétique de l'action enzymatique sur un dérivé estérifié de la bêta-lactoglobuline montre que la protéine estérifiée est hydrolysée très rapidement en solution aqueuse alors que la protéine native est insensible à une telle protéolyse.

L'invention rend possible l'hydrolyse de cette protéine qui, sans la modification décrite ne serait pas hydrolysée ; d'autre part, elle conduit à la création de sites de coupure non conventionnels pour l'enzyme utilisé.

Pour la bêta-lactoglobuline éthylée, 31 sites de coupure ont été identifiés Gln5-Thr6 ; Thr6-Met7; Leu10-Asp11 ; Asp11-Ile12 ; Gln13-Lys14 ; Trp19-Tyr20; Leu22-Ala23 ; Ser27-Asp28 ; Asp28-Ile29 ; Leu32-Asp33; Leu39-Arg40 ; Val41-Tyr42 ; Leu46-Lys47 ; Ile56-Leu57; Leu57-Leu58 ; Trp61-Glu62 ; Lys75-Thr76 ; Val81-Phe82; Phe82-Lys83 ; Leu87-Asn88 ; Glu89-Asn90 ; Val92-Leu93; Leu93-Val94 ; Asp98-Tyr99 ; Met107-Glu108 ; Leu117-Ala118 ; Asp130-Glu131 ; Leu133-Glu134 ; Phe136-Asp137; Asp137-Lys138 ; Leu149-Ser150.

### Exemple 2 : Hydrolyse pepsique de la bêta-caséine

La bêta-caséine A1 brute est préparée selon la méthode décrite par Zittle, C.A., Custer, J.H. (1963, j. Dairy Sci. **46**, 1183-1188). Le substrat est ensuite purifié par chromatographie selon la méthode de Mercier,J.C., Maubois, J.L. Poznanski, S., Ribadeau-Dumas, B. (1968, Bull. Soc. Chim. Biol. **50**, 521-530) sur une colonne Q-Sepharose fast flow (marque déposée), de la Société PHARMACIA, UPPSALA, SUEDE.

### Exemple 2a

### Estérification

La bêta-caséine estérifiée est préparée en utilisant une modification de la méthode décrite par Fraenkel-Conrat et Olcott (1945), citée supra. La bêta-caséine purifiée est dispersée dans l'éthanol pour obtenir une suspension à 2 %. Sous agitation, on ajoute doucement à la suspension protéine-alcool de l'acide chlorhydrique 12N pour obtenir une suspension 0,06-0,68N en acide. Le produit obtenu est maintenu sous agitation à 4°C pendant plusieurs jours en fonction du degré d'estérification désiré. Après séchage sous vide, les échantillons sont stockés à une température de -80°C. Un échantillon témoin a été préparé de la même manière sans acide chlorhydrique.

### Analyse des protéines estérifiées

La détermination du taux d'estérification de la bêta-caséine avec l'éthanol a été réalisée en utilisant la réaction colorée avec l'hydrochlorure d'hydroxylamine mise au point par Halpin et Richardson (1985), modifiée selon Bertrand Harb et autres (1991). Dans le cas présent, la bêta-caséine a été estérifiée à 55 %.

### Hydrolyse

La bêta-caséine et la bêta-caséine estérifiée (2 mg/ml) ont été dissoutes dans de l'acide citrique 20mM, pH2,6. La pepsine (1 mg/ml d'H₂O en concentration initiale) a été ajoutée dans un rapport enzyme/substrat protéique (E/S) de 0,2 %. Le mélange obtenu a été incubé à 20°C. Des échantillons ont été prélevés à 1, 2, 4, 20 et 40 Heures ; l'hydrolyse a été stoppée par addition de 1,5 volume d'un tampon Tris HCl 0,2M, pH8,0.

### Exemple 2b

Une estérification similaire a été conduite
sur la bêta-caséine avec du méthanol à la place de l'éthanol ; l'hydrolyse postérieure a été réalisée sur la bêta-caséine méthylée, d'une façon identique à celle de la caséine bêta éthylée décrite ci-avant. Pour cet exemple également, la bêta-caséine a été estérifiée à 55 %.

### Résultats

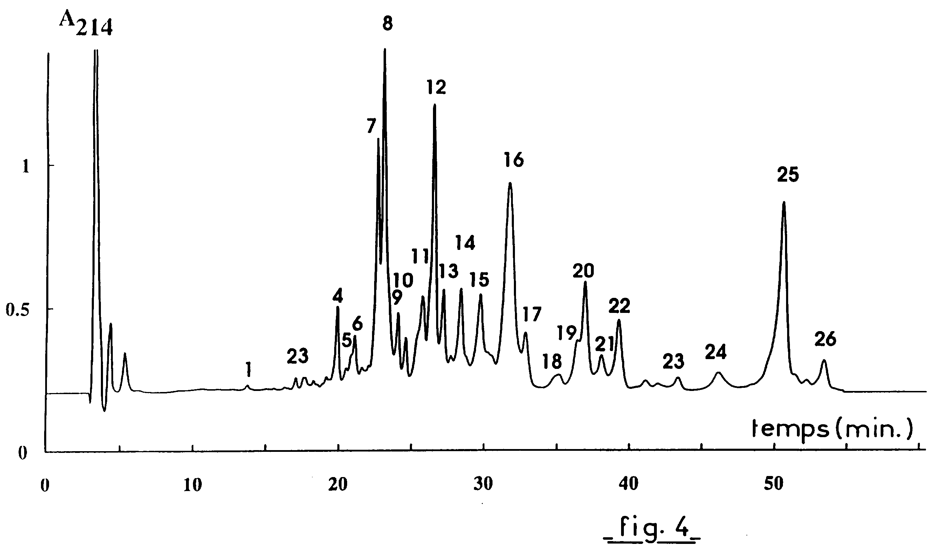

La figure 4 montre le profil chromatographique CLHP d'un hydrolysat pepsique de caséine bêta native après 10 Heures d'hydrolyse.

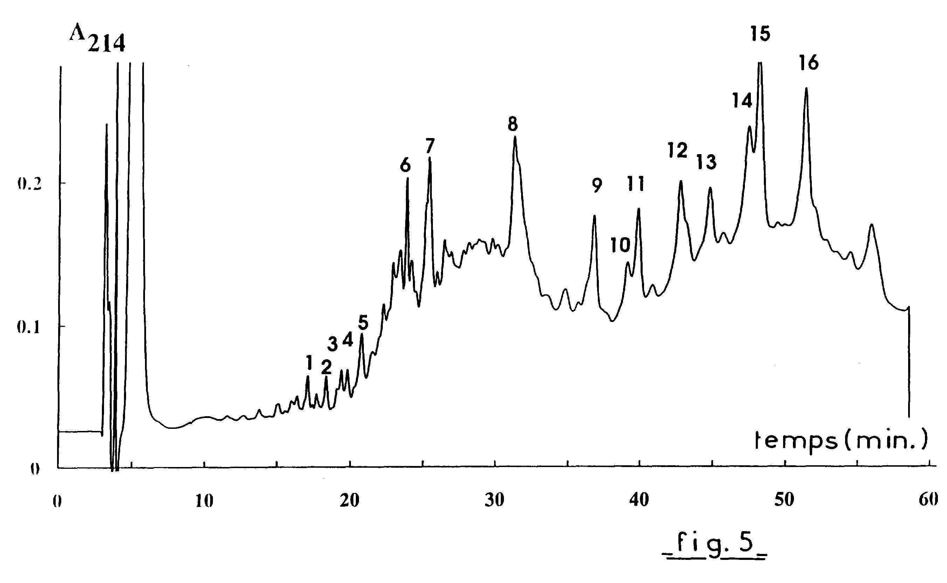

La figure 5 montre le profil chromatographique CLHP (après 10 heures d'hydrolyse) d'un hydrolysat pepsique de caséine bêta méthylée (ester), préparé selon l'exemple 2b.

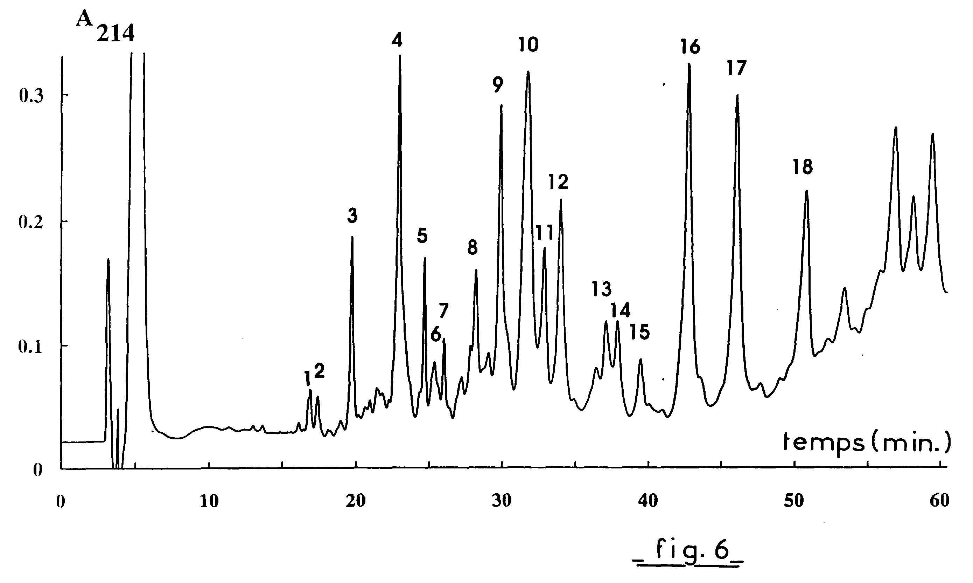

La figure 6 montre le profil chromatographique CLHP (après 10 heures d'hydrolyse) d'un hydrolysat pepsique de caséine bêta éthylée (ester), préparé selon l'exemple 2a.

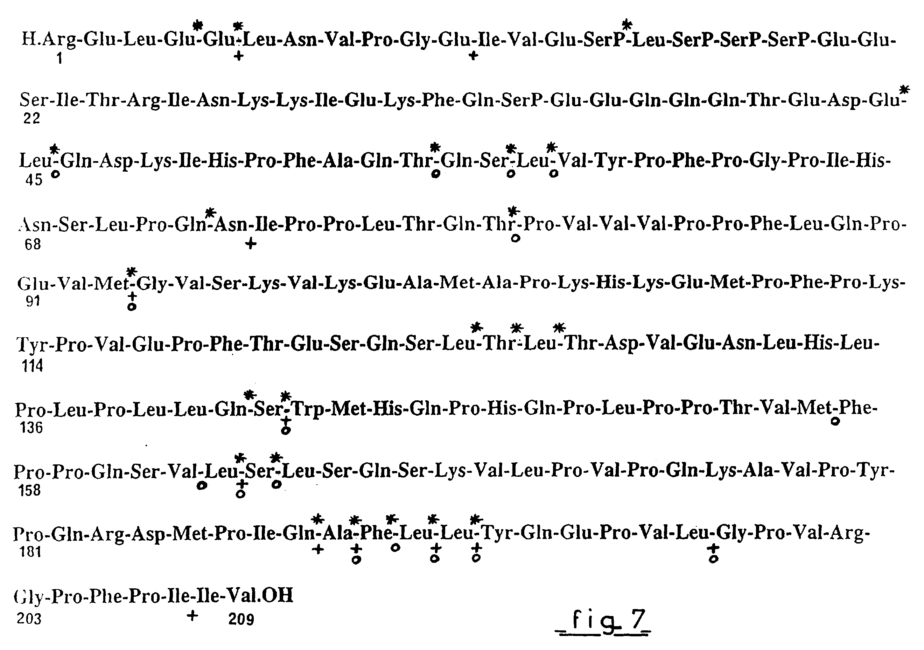

La figure 7 montre la structure primaire de la caséine bêta A1 avec les sites de coupure pepsique au sein de la caséine bêta native (*), au sein de la caséine bêta méthylée (+), et au sein de la caséine bêta éthylée (o).

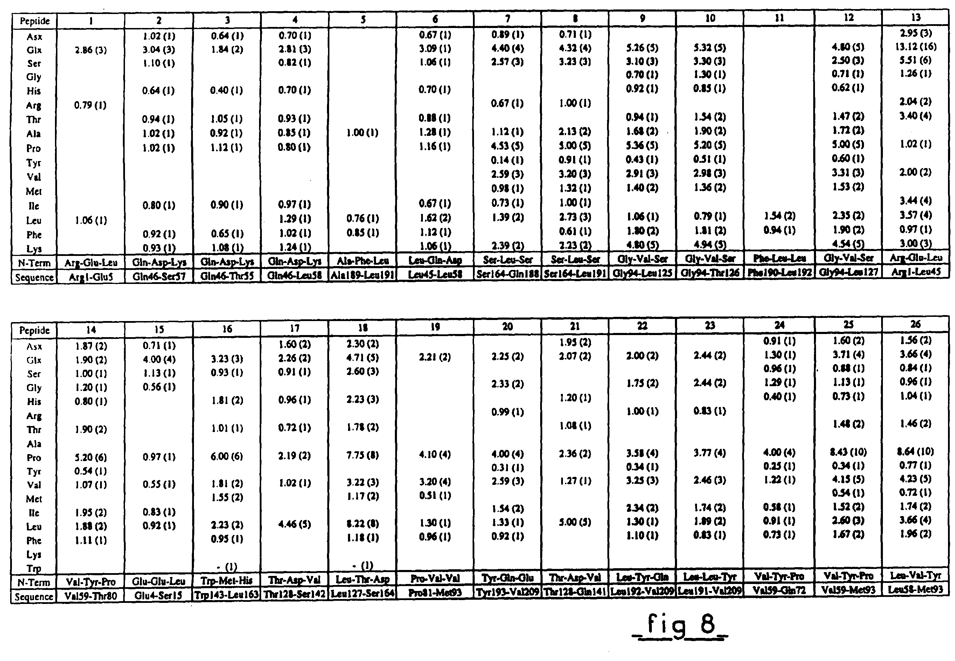

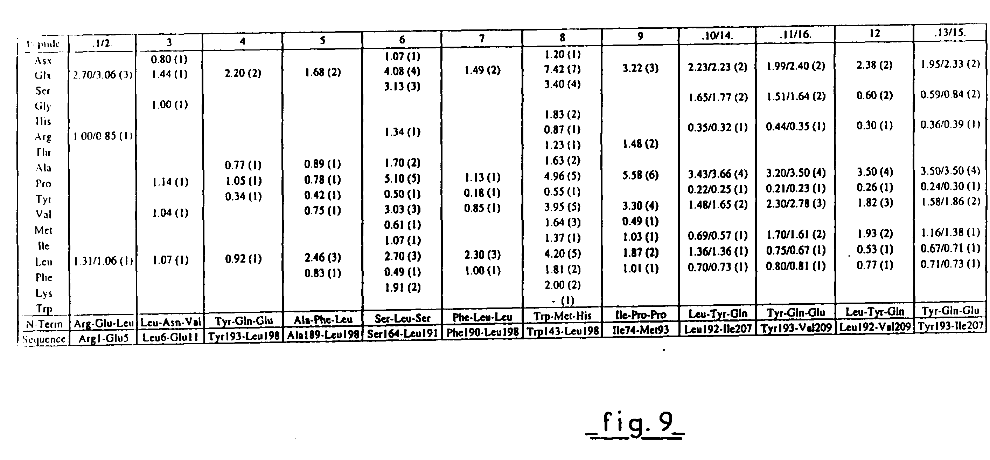

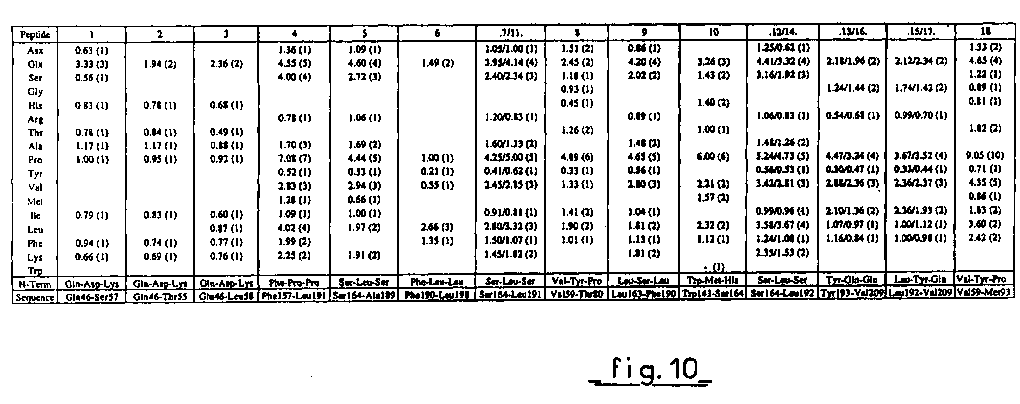

Les figures 8, 9 et 10 montrent, respectivement et sous forme de tableaux, les compositions en acides aminés et séquences N-terminales des peptides pepsiques de la caséine bêta native, de la caséine bêta méthylée (ester) et de la caséine bêta éthylée (ester).

Dans le cas de la caséine bêta estérifiée, six sites de coupure nouveaux ont été identifiés : Glu11-Ile12 ; Asn73-Ile74 ; Met156-Phe157 ; Val162-Leu163; Leu198-Gly199 ; Ile207-Ile208.

### Exemple 3 : Hydrolyse trypsique de la bêta- lactoglobuline

La bêta-lactoglobuline et la bêta-lactoglobuline estérifiée sont préparées selon l'exemple 1 et hydrolysées par la trypsine (trypsine bovine traitée TPCK 10,000-13,000 U/mg ; de la Société Sigma Chimie).

La bêta-lactoglobuline et la bêta-lactoglobuline estérifiée (2 mg/ml) sont dissoutes dans un tampon TrisHCl, 0,2M, de pH8,0. La trypsine, auparavant solubilisée dans HCl 0,01N, est ajoutée dans un rapport enzyme/substrat protéique (E/S) de 2,5 %. Le mélange obtenu est incubé à 37°C et l'hydrolyse est stoppée après 24 Heures par l'addition de 0,5 volume d'HCl 0,2N.

### Résultats

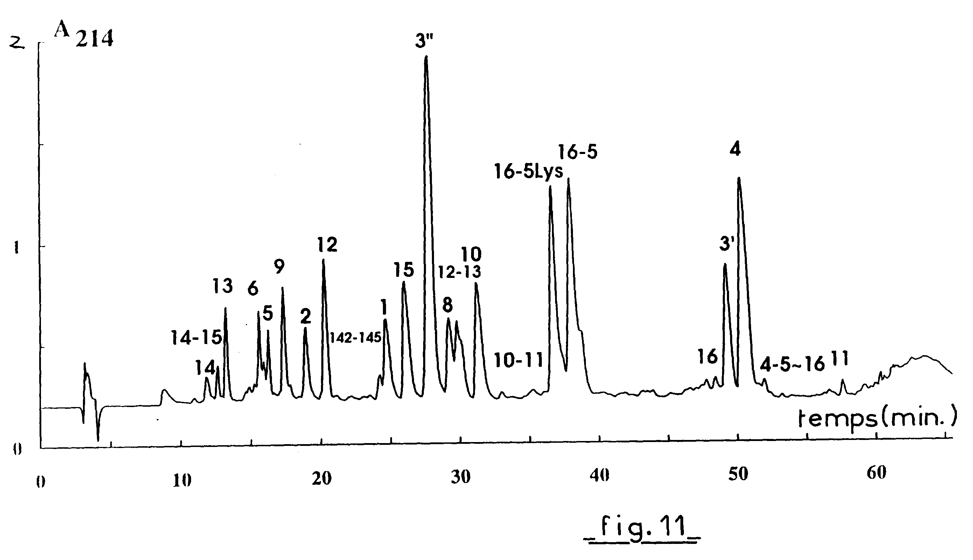

La figure 11 montre le profil chromatographique CLHP d'un hydrolysat trypsique de la bêta-lactoglobuline native (après 24 heures d'hydrolyse).

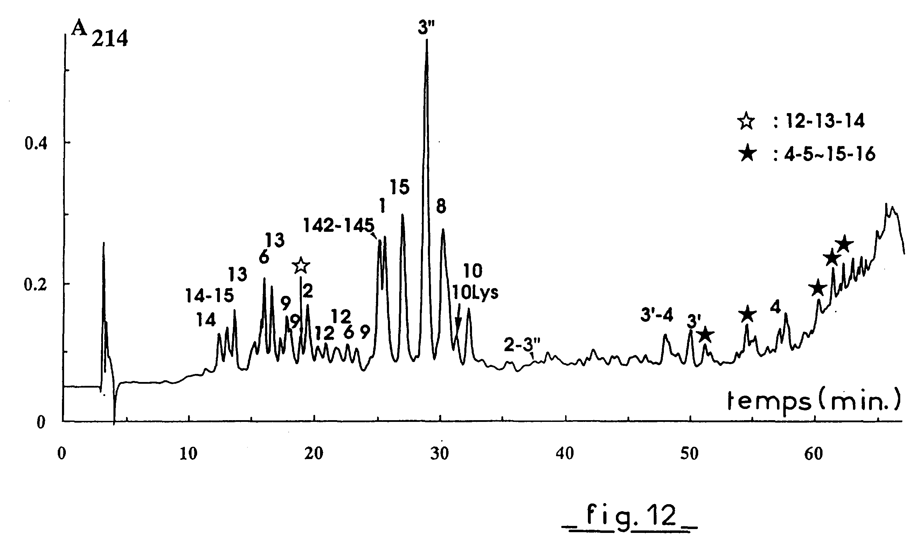

La figure 12 montre le profil chromatographique CLHP (après 24 heures d'hydrolyse) d'un hydrolysat trypsique de la bêta-lactoglobuline éthylée (ester).

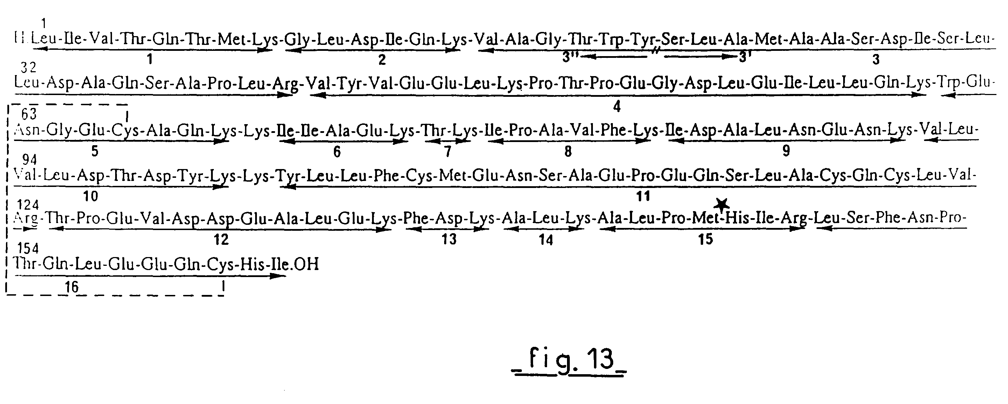

La figure 13 montre la structure primaire de la bêta-lactoglobuline B ; on y indique les 16 peptides trypsiques obtenus et le site de clivage atypique (*) de la bêta-lactoglobuline estérifiée.

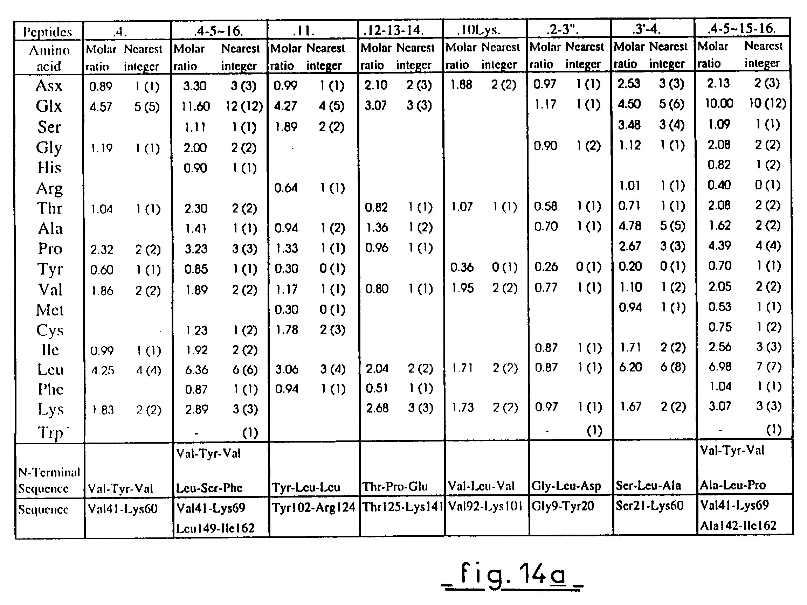

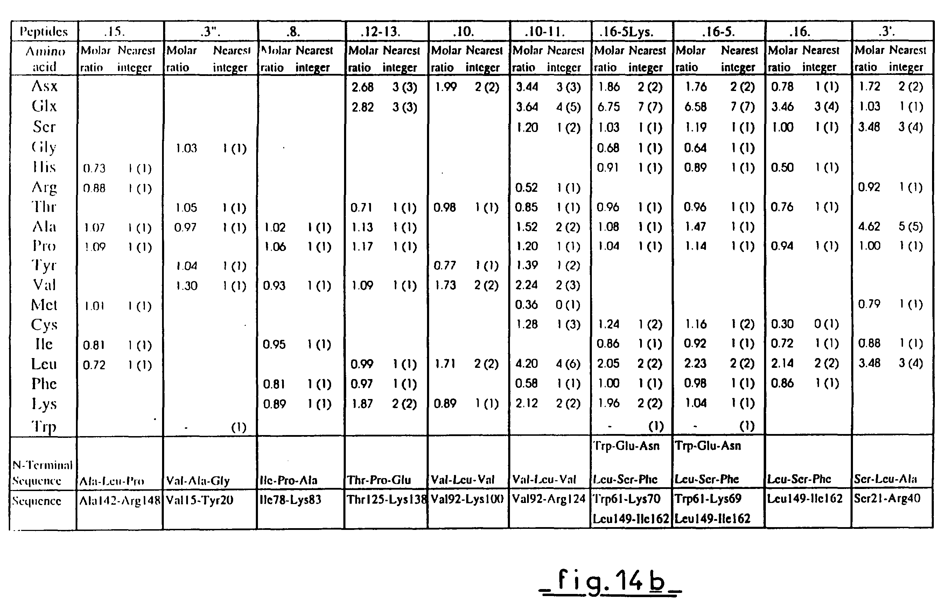

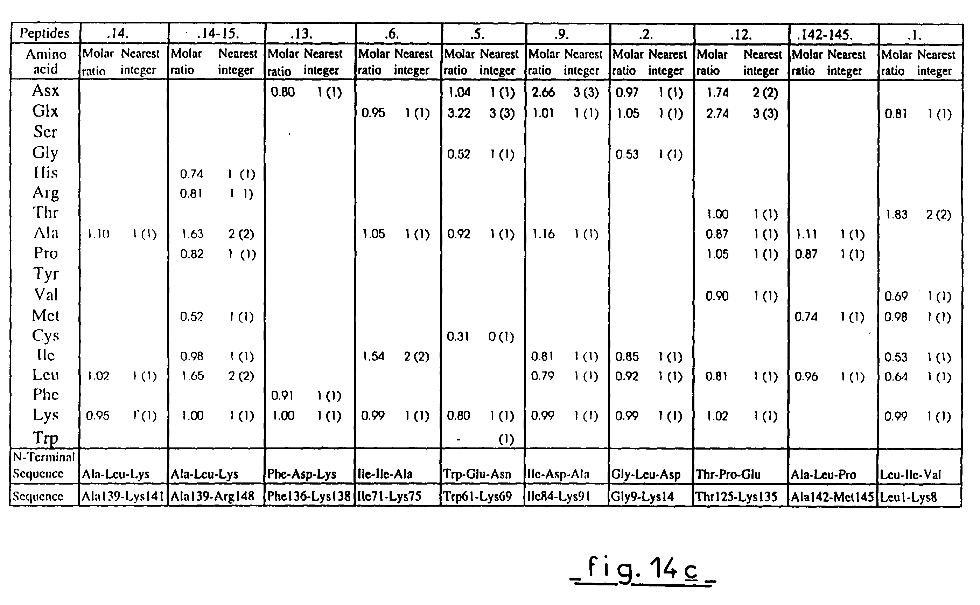

Les figures 14a, 14b et 14c montrent, sous forme de tableaux, la composition en acides aminés et séquences N-terminales des peptides trypsiques de la bêta-lactoglobuline native et de la bêta-lactoglobuline éthylée.

L'analyse des peptides trypsiques de la bêta-lactoglobuline montre que l'estérification n'empêche aucune liaison cible d'être coupée même quand un résidu aspartyle ou glutamyle estérifié est au voisinage de la liaison cible, ce qui conduit à l'obtention de peptides estérifiés. Un site de coupure atypique a été mis en évidence : Met145-His146.

### Exemple 4 : Hydrolyse trypsique de la bêta-caséine

La bêta-caséine et la bêta-caséine estérifiée obtenues selon l'exemple 2 ont été soumises à l'action de la trypsine (trypsine de bovin traitée TPCK ; 10,000-13,000 U/mg).

La bêta-caséine native et la bêta-caséine estérifiée (2 mg/ml) sont dissoutes dans un tampon Tris-HCl 0,2M, de pH8,0. La trypsine auparavant solubilisée dans HCl 0,01N est ajoutée dans un rapport enzyme/substrat protéique (E/S) de 1,25 %. Le mélange obtenu est incubé à 20°C ; l'hydrolyse est stoppée après 24 Heures par addition de 0,5 volume d'HCl 0,2N.

### Résultats

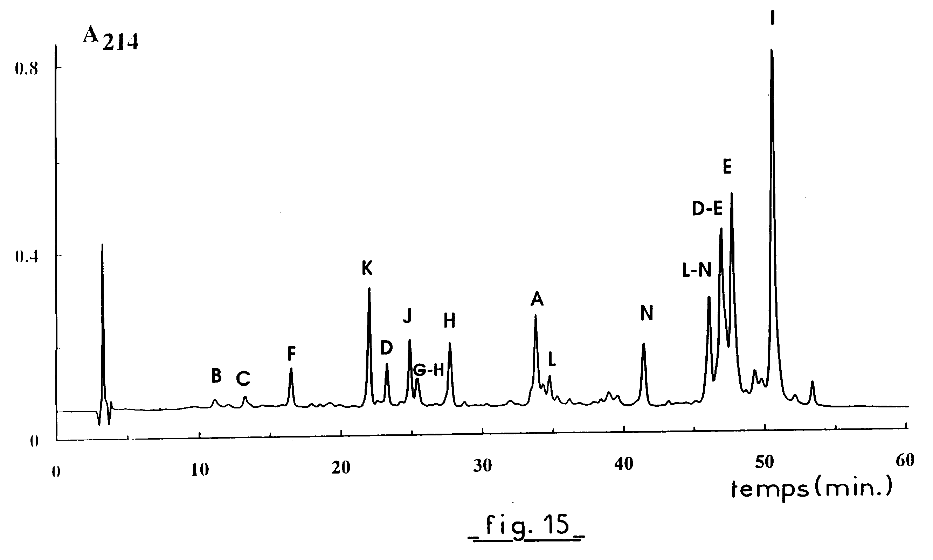

La figure 15 montre le profil chromatographique CLHP d'un hydrolysat trypsique de caséine bêta native (après 24 heures d'hydrolyse).

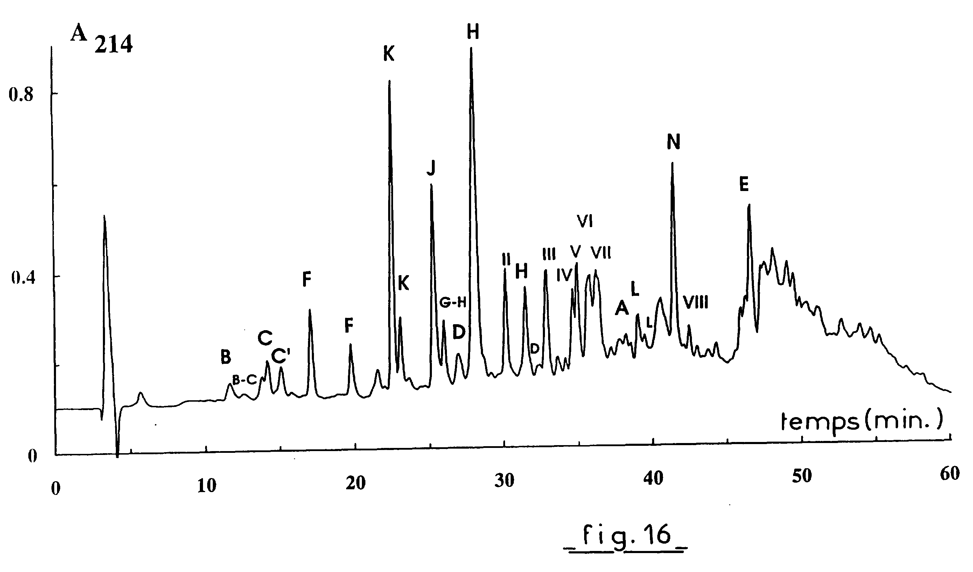

La figure 16 montre le profil chromatographique CLHP (après 24 heures d'hydrolyse) d'un hydrolysat trypsique de caséine bêta méthylée (ester).

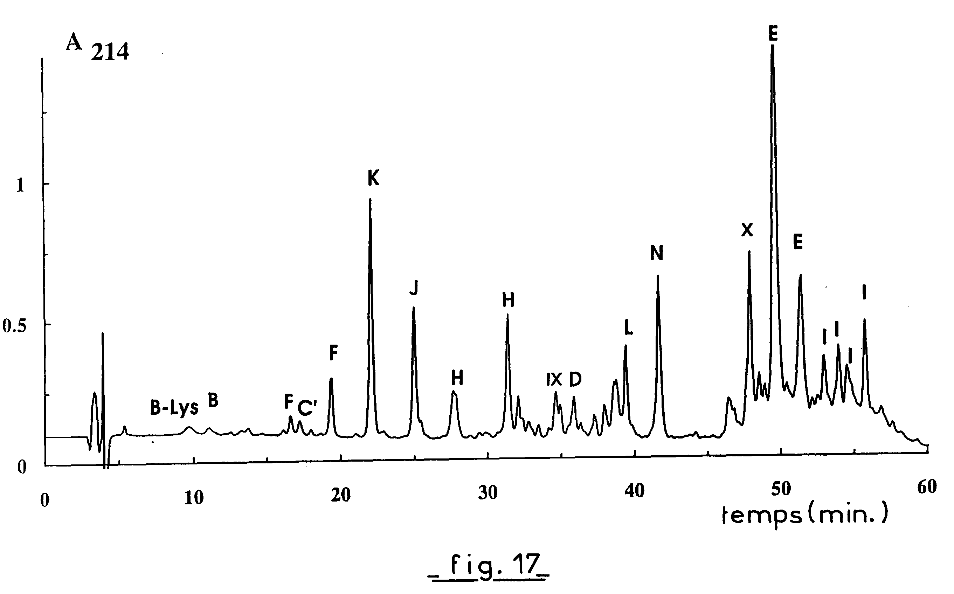

La figure 17 montre le profil chromatographique CLHP (après 24 heures d'hydrolyse) d'un hydrolysat trypsique de caséine bêta éthylée (ester).

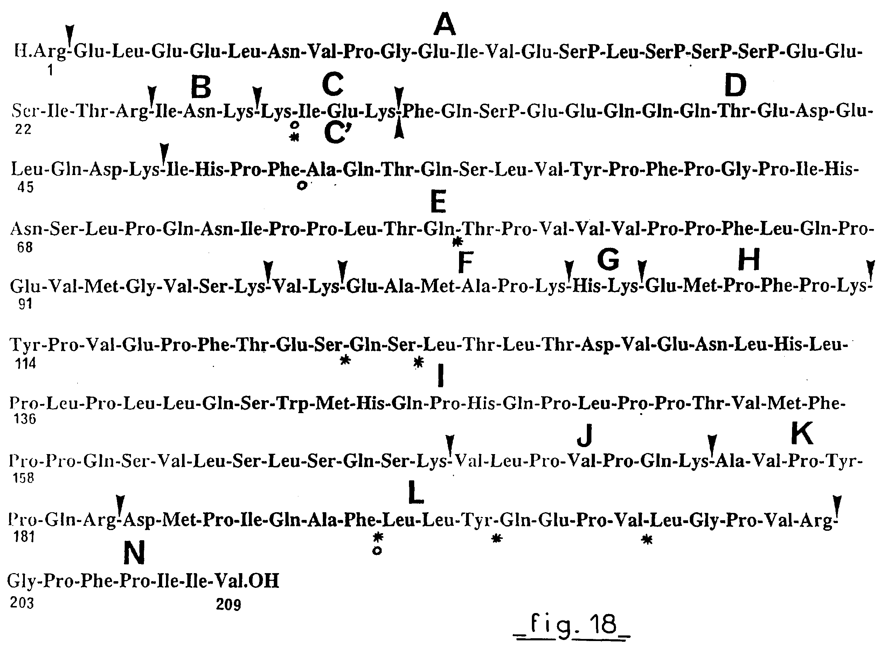

La figure 18 montre la structure primaire de la caséine bêta A1 ; les lettres A à N indiquent les peptides trypsiques de la caséine bêta native et on a indiqué les sites de clivage atypique : pour la caséine bêta éthylée (o) et pour la caséine bêta méthylée (*).

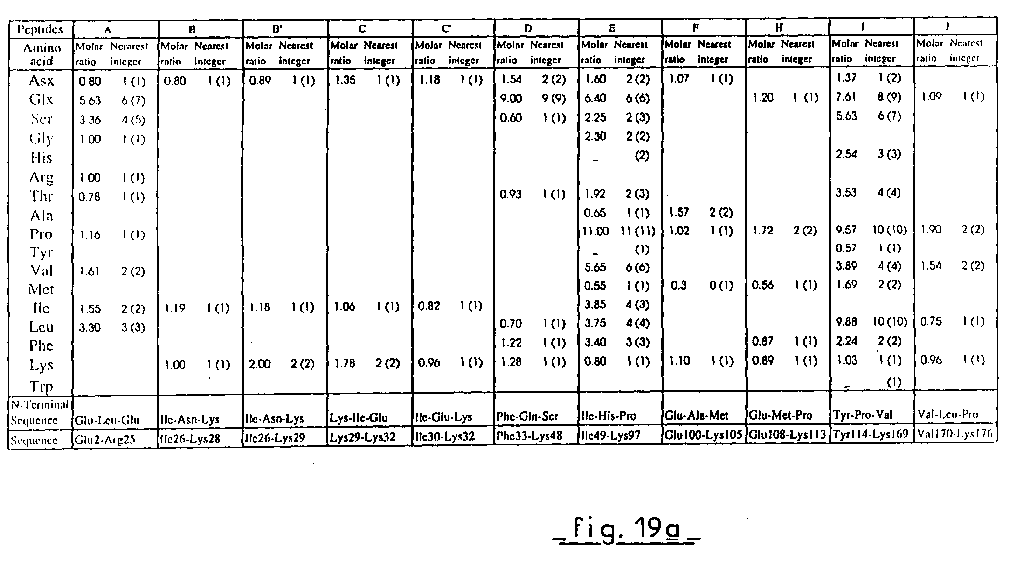

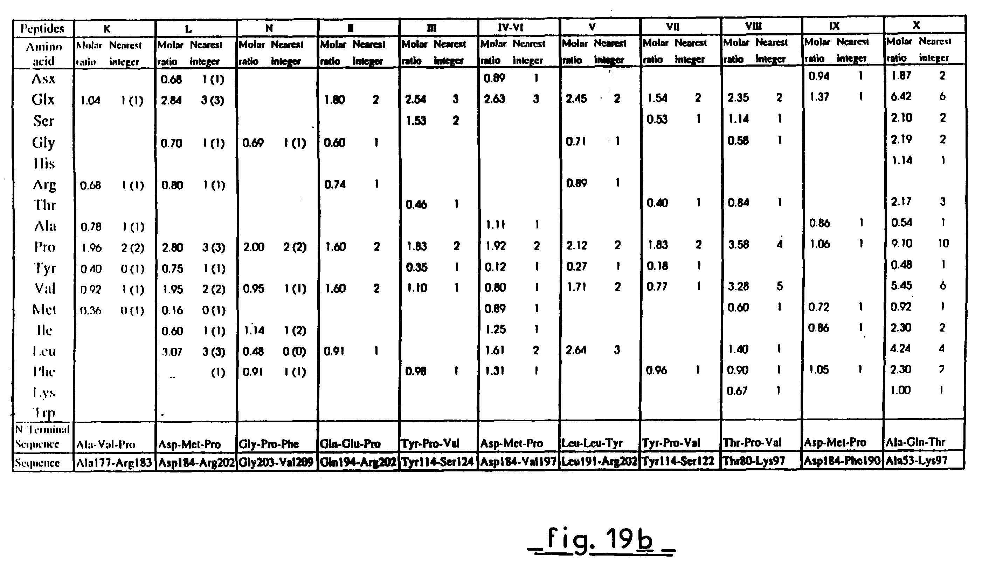

Les figures 19a et 19b montrent, sous forme de tableaux, la composition en acides aminés et séquences N-terminales des peptides trypsiques de la caséine bêta native, de la caséine bêta méthylée et de la caséine bêta éthylée.

Comme pour la bêta-lactoglobuline, l'analyse des peptides trypsiques de la caséine bêta montre que l'estérification n'empêche aucune liaison cible d'être coupée, même quand un résidu aspartyle ou glutamyle estérifié est au voisinage de la liaison cible, ce qui conduit à l'obtention de peptides estérifiés. Des sites de coupure atypique ont été mis en évidence : Phe52-Ala53 ; Gln79-Thr80 ; Ser122-Gln123 ; Ser124-Leu125 ; Phe190-Leu191 ; Tyr193-Gln194 ; Val197-Leu198.

## Revendications

1. Procédé de traitement d'un substrat d'origine animale ou végétale contenant au moins une protéine ou un peptide doté d'au moins un groupe estérifiable en plus du groupe carboxylique C-terminal de l'enchaînement, par une technique d'hydrolyse enzymatique conduisant à l'obtention de produits peptidiques, caractérisé en ce qu'il consiste, avant l'opération d'hydrolyse enzymatique, à soumettre ledit substrat protéique à une réaction d'estérification d'au moins un groupe estérifiable de ladite protéine ou dudit peptide, en plus de l'estérification éventuelle du groupe carboxylique C-terminal de l'enchaînement, la succession desdites opérations d'estérification et d'hydrolyse permettant l'obtention de peptides dont certains au moins sont estérifiés au niveau de l'un au moins de leurs groupes estérifiables, à l'exception de leur groupe carboxylique C-terminal.

2. Procédé selon la revendication 1, caractérisé en ce qu'il consiste à réaliser la réaction d'estérification sur au moins un groupe carboxylique de la protéine ou du peptide.

3. Procédé selon la revendication 2, caractérisé en ce que l'estérification de la protéine ou du peptide est réalisée en présence d'un alcool.

4. Procédé selon la revendication 3, caractérisé en ce que l'estérification est réalisée en présence d'un alcool aliphatique possédant entre 1 et 5 atomes de carbone.

5. Procédé selon l'une quelconque des revendications 1 à 4, caractérisé en ce que la réaction d'estérification est précédée d'une hydrolyse partielle de la protéine.

6. Procédé selon l'une quelconque des revendications 1 à 5, caractérisé en ce que la réaction d'estérification est suivie d'une succession d'opérations d'hydrolyse au moyen de différentes enzymes protéolytiques ou peptidasiques.

7. Procédé selon l'une quelconque des revendications 1 à 6, caractérisé en ce qu'on utilise un substrat protéique d'origine laitière contenant notamment de la bêta-caséine ou de la bêta-lactoglobuline.

## Claims

1. Treatment method of an animal or vegetable substrate comprising at least a protein or a peptide provided with at least an esterifiable group in addition of the C-terminal carboxylic group of the sequence by an enzymatic hydrolysis giving peptides, characterized in that before the enzymatic hydrolysis an esterification reaction with at least an esterifiable group of the said protein or peptide is carried out on the said protein substrate in addition of the optional esterification of the C-terminal carboxylic group of the sequence, the making of said esterification and hydrolysis steps giving peptides at least some of which are esterified in at least one of the esterifiable groups thereof except the C-terminal carboxylic group thereof.

2. Method according to claim 1, characterized in that it consists of carrying out the esterification reaction on at least one carboxylic group of the protein or peptide.

3. Method according to claim 2, characterized in that the esterification of the protein or the peptide is carried out in the presence of an alcohol.

4. Method according to claim 3, characterized in that the esterification is carried out in the presence of an aliphatic alcohol having between one and five carbon atoms.

5. Method according to any one of claims 1 to 4, characterized in that the esterification reaction is preceded by partial hydrolysis of the protein.

6. Method according to any one of claims 1 to 5, characterized in that the esterification reaction is followed by a succession of hydrolysis operations using different proteolytic or peptidase enzymes.

7. Method according to any one of claims 1 to 6, characterized in that a milk protein substrate is used containing in particular beta-casein or beta-lactoglobulin.

## Patentansprüche

1. Verfahren zur Behandlung eines Substrats tierischen oder pflanzlichen Ursprungs, das wenigstens ein Protein oder ein Peptid, das mit wenigstens einer veresterungsfähigen Gruppe außer der C-terminalen Carboxygruppe der Kette versehen ist, enthält, durch eine enzymatische Hydrolysetechnik, die dazu führt, daß man peptidische Produkte erhält, dadurch gekennzeichnet, daß es darin besteht, daß man vor der enzymatischen Hydrolyseoperation das Proteinsubstrat einer Veresterungsreaktion mit wenigstens einer veresterungsfähigen Gruppe des Proteins oder des Peptids außer der eventuellen Veresterung der C-terminalen Carboxygruppe der Kette unterzieht, wobei die Abfolge der Operationen der Veresterung und der Hydrolyse es erlaubt, Peptide zu erhalten, von denen wenigstens einige an wenigstens einer ihrer veresterungsfähigen Gruppen verestert sind, mit Ausnahme ihrer C-terminalen Carboxygruppe.

2. Verfahren gemäß Anspruch 1, dadurch gekennzeichnet, daß es darin besteht, daß man die Veresterungsreaktion mit wenigstens einer Carboxygruppe des Proteins oder des Peptids durchführt.

3. Verfahren gemäß Anspruch 2, dadurch gekennzeichnet, daß die Veresterung des Proteins oder des Peptids in Gegenwart eines Alkohols erfolgt.

4. Verfahren gemäß Anspruch 3, dadurch gekennzeichnet, daß die Veresterung in Gegenwart eines aliphatischen Alkohols erfolgt, der zwischen 1 und 5 Kohlenstoffatome enthält.

5. Verfahren gemäß einem der Ansprüche 1 bis 4, dadurch gekennzeichnet, daß der Veresterungsreaktion eine partielle Hydrolyse des Proteins vorausgeht.

6. Verfahren gemäß einem der Ansprüche 1 bis 5, dadurch gekennzeichnet, daß der Veresterungereaktion eine Abfolge von Hydrolyseoperationen mittels verschiedener proteolytischer oder peptidasischer Enzyme folgt.

7. Verfahren gemäß einem der Ansprüche 1 bis 6, dadurch gekennzeichnet, daß ein Proteinsubstrat verwendet wird, das aus der Milch stammt und das insbesondere β-Casein oder β-Lactoglobulin enthält.
